# EUROPEAN PATENT APPLICATION

(11) **EP 4 435 091 A1**
(43) Date of publication of application: **25.09.2024**
(21) Application number: 23181175.3
(22) Date of filing: 23.06.2023
(51) Int. Cl.: C12N 1/20, A23L 33/135, A61K 35/744, C12R 1/46

(54) **NOVEL STREPTOCOCCUS SALIVARIUS STRAIN HAVING ANTIBACTERIAL, ANTIFUNGAL, ANTI-INFLAMMATORY ACTIVITY AND INHIBITING DENTAL CARIES AND ORAL COMPOSITION COMPRISING THE SAME**

(30) Priority: 23.03.2023 KR 20230038272
(71) Applicant: Greenstore Inc., Seongnam-si Gyeonggi-do 13558 (KR)
(72) Inventor: PARK, Young Chang, 13528 Gyeonggi-do (KR); BANG, Chaeyoung, 05618 Seoul (KR); LEE, Sung Hoon, 31115 Chungcheongnam-do (KR); BAEK, Dong Heon, 06085 Seoul (KR)
(74) Representative: Kraus & Lederer PartGmbB

(57) **Abstract**

Disclosed are a novel *Streptococcus salivarius* (*S. salivarius* G7, KCCM13161P) strain, a composition containing the same for prevention or alleviation of an oral disease, and oral probiotics, and specifically, the novel *Streptococcus salivarius* (*S. salivarius* G7, KCCM13161P) strain can prevent, treat, and alleviate periodontal diseases through strong antibacterial and antiinflammatory activities against periodontal disease-causing strains, prevents, treat, and alleviates dental caries through strong antibacterial activity against dental caries-causing strains and biofilm formation inhibitory ability, and suppress halitosis caused by oral diseases, thereby contributing to oral health improvement.

## Description

### CROSS-REFERENCE TO RELATED APPLICATION(S)

This application is based on and claims priority under 35 U.S.C. 119 to Korean Patent Application No. 10-2018-0000000, filed on January 00, 2018, in the Korean Intellectual Property Office, the disclosure of which is herein incorporated by reference in its entirety.

### BACKGROUND OF THE INVENTION

### 1. Field of the invention

The present disclosure relates to a novel *Streptococcus salivarius* strain having antibacterial, antifungal, and antiinflammatory activities and dental caries inhibitory activity and an oral composition containing the same.

### 2. Description of the Prior Art

Infectious diseases in the oral cavity may be classified into gingival and dental diseases, and these infectious diseases are related to biofilms formed of by multiple species of bacteria. Biofilms of healthy persons are formed of balanced multiple species of bacteria with a high proportion of commensal bacteria. However, the proliferation of particular bacteria due to the collapse of such a balance results in oral diseases.

*Actinomyces israelii* and *Actinomyces naeslundii* are typical causative bacteria of gingivitis, and *Streptococcus mutans* and *Streptococcus sobrinus* are known as cariogenic bacteria (Howell A. et al., 1962; and Loesche WJ. et al., 1978). These bacteria have acidogenesis and aciduricity, causing dental caries (Conrads G et al., 2014). *Enterococcus faecalis* is a dominant species of human Enterococci and is involved in oral diseases, such as root canal infection, periodontitis, and peri-implantitis, due to its antibiotic resistance (Dahlen G., et al., 1993; and Rams TE., et al., 2013).

Some strains may be used as probiotics for oral use (Li X., 2021; and Hale JDF., et al., 2022). These bacteria suppress neighboring bacteria by producing bacteriocin during the competition for habitats in bacteria. *Streptococcus salivarius* also produces various types of lantibiotics, such as salivaricin A, salivaricin B, salivaricin 9, and salivaricin G3216-18, which are similar to bacteriocin (Wescombe PA., et al., 2006). *Streptococcus salivarius,* which is a gram-positive facultative anaerobic bacterium, acts as a commensal bacterium that inhabits the surface of human mucous membranes (Aas JA., et al., 2005) and plays important ecological roles, such as forming barriers against pathogens and reducing adhesion and colonization (Wescombe PA., et al., 2006), and thus its possibility of use as oral probiotics is constantly being raised.

Recently, various research papers have published the results showing that harmful bacteria present in the oral cavity adversely affect systemic diseases (lupus, systemic sclerosis, inflammatory bowel disease, dementia, etc.), and this has been expanding to the concept that maintaining the oral microbial balance can influence dental health as well as systemic health.

Most systemic diseases are known to be caused by an imbalance of the normal bacterial flora in the human body or by the invasion and proliferation of foreign microbes. Therefore, using oral probiotics capable of maintaining the microbial ecosystem while also having an antibacterial effect rather than using antibiotics inhibiting the growth of microorganisms can help improve public health.

Accordingly, the development of oral probiotics having a wide antibacterial activity is urgently needed.

### [Prior Art Documents]

### [Patent Documents]

(Patent Document 0001) Korean Patent Publication No. 10-2022-0139280, Oral composition comprising novel strain of Lactobacillus plantarum, Published 15 October 2022

(Patent Document 0002) Korean Patent Registration No. 10-2198552, Composition improving, preventing or treating halitosis or dental caries comprising Lactobacillus fermentum OK derived from human oral cavity, Registered 29 December 2020

(Patent Document 0003) Korean Patent Publication No. 10-2023-0017372, Composition comprising superoxide dismutase and/or bacillus strain spores, and uses thereof for improving oral health, Published 03 February 2023

(Patent Document 0004) Korean Patent Registration No. 10-2198553, Composition improving, preventing or treating halitosis or dental caries comprising Lactobacillus gasseri HHuMIN D derived from human oral cavity, Registered 29 December 2020

### [Non-Patent Documents]

(Non-Patent Document 0001) Howell A, Stephan RM, Paul F. Prevalence of Actin-omyces israelii, A. naeslundii, Bacterionema matruchotii, and Candida albicans in selected areas of the oral cavity and saliva. J Dent Res 1962;41:1050-1059.

(Non-Patent Document 0002) Loesche WJ, Syed SA. Bacteriology of human experimental gingivitis: effect of plaque and gingivitis score. Infect Immun 1978;21:830-839.

(Non-Patent Document 0003) Conrads G, de Soet JJ, Song L, Henne K, Sztajer H, Wagner-Dobler I, et al., Comparing the cariogenic species Streptococcus sobrinus and S. mutans on whole genome level. J Oral Microbiol 2014;6:26189.

(Non-Patent Document 0004) Dahlen G. Role of suspected periodontopathogens in microbiological monitoring of periodontitis. Adv Dent Res 1993;7:163-174.

Rams TE, Feik D, Mortensen JE, Degener JE, Winkelhoff AJ. Antibiotic susceptibility of periodontal Enterococcus faecalis. J Periodontol 2013;84:1026-1033.

(Non-Patent Document 0005) Aas JA, Paster BJ, Stokes LN, Olsen I, Dewhirst FE. Defining the normal bacterial flora of the oral cavity. J Clin Microbiol 2005;43:5721-5732.

(Non-Patent Document 0006) Wescombe PA, Burton JP, Cadieux PA, Klesse NA, Hyink O, Heng NC, et al. Megaplasmids encode differing combinations of lantibiotics in Streptococcus salivarius. Antonie Van Leeuwenhoek 2006;90:269-280.

(Non-Patent Document 0007) Li X, Fields FR, Ho M, Marshall-Hudson A, Gross R, Casser ME, et al. Safety assessment of Streptococcus salivarius DB-B5 as a probiotic candidate for oral health. Food Chem Toxicol 2021;153:112277.

(Non-Patent Document 0008) Hale JDF, Jain R, Wescombe PA, Burton JP, Simon RR, Tagg JR. Safety assessment of Streptococcus salivarius M18 a probiotic for oral health. Benef Microbes 2022;13:47-60.

(Non-Patent Document 0009) Wescombe PA, Upton M, Renault P, Wirawan RE, Power D, Burton JP, Chilcott CN, Tagg JR. Salivaricin 9, a new lantibiotic produced by Streptococcus salivarius. Microbiology 2011;157:1290-1299.

### SUMMARY OF THE INVENTION

An aspect of the present disclosure is to provide a novel *Streptococcus salivarius* strain having antibacterial, antifungal, and antiinflammatory effects and an oral composition containing the same.

In accordance with an aspect of the present disclosure, there is provided a novel *Streptococcus salivarius* strain *(S. salivarius* G7, KCCM13161P) having antibacterial, antifungal, and antiinflammatory activities.

The antibacterial activity may include an antibacterial activity against at least one selected from the group consisting of *Aggregatibacter actinomycetemcomitans, Actinomyces israelii, Actinomyces naeslundii, Enterococcus faecalis, Fusobacterium nucleatum, Porphyromonas gingivalis, Prevotella intermedia, Bacteroides intermedius, Prevotella nigrescens, Streptococcus mutans, Streptococcus sobrinus, Streptococcus pneumoniae, Streptococcus pyogenes, Actinomyces viscosus, Escherichia coli,* and *Staphylococcus aureus,* but is not limited thereto.

Herein, *"Aggregatibacter actinomycetemcomitans"* is one of the representative gingivitis-causing bacteria and is a gram-negative, facultative anaerobic non-motile bacterium related with locally aggressive periodontitis. Less frequently, *A. actinomycetemcomitans* causes non-oral infections such as endocarditis, and produces leukotoxin (LT) and cytolethal distending toxin (Cdt) as exotoxins. The exotoxin LT forms pores in the plasma membrane of monocytes, neutrophils, and some lymphocytes to damage cells, thereby inhibiting immunity. The exotoxin Cdt is a DNA degrading enzyme, and inhibits cell division of several cells including epithelial cells, gingival fibroblasts, and lymphocytes to induce apoptosis in some cells with cell division suppressed. The strain secretes lipopolysaccharide (LPS) as an endotoxin to induce macrophage pro-inflammatory cytokines (IL-1 and TNF) production and induce bone resorption and collagen destruction.

Herein, *"Actinomyces israelii"* lives commensally in the vagina, colon, and mouth of normal persons, and is an opportunistic pathogen to cause an infectious disease when the individual's immunity is low, causing gingivitis. Actinomycosis is most frequently caused by *A*. *israelii,* and can be treated by an antibiotic agent.

Herein, *"Actinomyces naeslundii"* is a gram-positive rod-shaped bacterium found in women with bacterial vaginosis and in the mouth of humans, wherein the strain is associated with various periodontal diseases including tooth decay and causes, particularly, gingivitis. *A. naeslundii* also causes actinomycosis, and may form abscesses secreting edema and pus and may be accompanied by tissue fibrosis. *A. naeslundii* frequently infects the mouth, face, and neck, but unusually infects the chest, abdomen, pelvis, and central nervous system.

Herein, *"Enterococcus faecalis"* is a gram-positive, commensal bacterium that inhabits the gastrointestinal tract of humans and other mammals and is one of the representative bacteria causing periapical inflammation. The strain has been frequently found in the root canal-treated teeth in prevalence values ranging from 30% to 90% of the cases, and re-infected root canal-treated teeth are about nine times more likely to harbor *E. faecalis* than cases of primary infections. Rarely, the strain may cause endocarditis, sepsis, urinary tract infection (UTI), meningitis, and other infections in humans, and is known to have multi-drug resistance (MRD) against aminoglycoside, aztreonam, cephalosporin, clindamycin, semi-synthetic penicillin, nafcillin and oxacillin, and trimethoprim/sulfamethoxazole, which are frequently used antibacterial agents.

Herein, *"Fusobacterium nucleatum"* is a gram-negative, anaerobic oral bacterium, commensal to the human oral cavity, and is one of the representative bacterial causing periodontitis, wherein the strain is a key component of periodontal plaque due to its ability to aggregate with other bacteria species in the oral cavity. Furthermore, many studies verified that the strain was associated with preterm birth and colorectal cancer.

Herein, *"Porphyromonas gingivalis"* is a non-motile, gram-negative, rod-shaped, anaerobic, pathogenic bacterium and a representative periodontitis-causing bacterium. *P*. *gingivalis* is found in the oral cavity with periodontal disease, the upper gastrointestinal tract, the respiratory tract, and the colon and is known to be associated with Alzheimer's disease and rheumatoid arthritis.

Herein, *"Prevotella intermedia (Bacteroides intermedius)"* is a gram-negative, obligate anaerobic pathogenic bacterium involved in periodontal infections, including gingivitis and periodontitis, and often found in acute necrotizing ulcerative gingivitis. *P. intermedia* uses steroid hormones as growth factors, so their numbers are higher in pregnant women and has also been isolated from women with bacterial vaginosis.

Herein, *"Prevotella nigrescens"* is a gram negative bacterium, a part of the normal oral flora, and one of the opportunistic periodontitis-causing bacteria, and is known to be associated with nasopharyngeal and intra-abdominal infections and carotid atherosclerosis in addition to periodontal diseases.

Herein, *"Actinomyces viscosus"* is a gram-positive, facultatively anaerobic bacterium that colonizes the mouths of 70% of adult humans. *A. viscosus* causes periodontal disease in animals, may cause dental calculus and root surface caries as well as endocarditis in humans, and very rarely, may cause lung infections.

Herein, *"Streptococcus mutans"* is also known as a tooth decay bacterium and is a main causing strain of tooth decay, together with Lactobacillus. *S. mutans* causes dental caries, which causes teeth to turn black and decay.

Herein, *"Streptococcus sobrinus"* is a gram-positive, non-motile, and anaerobic bacterium, and accelerates the formation of dental caries by combination with *Streptococcus mutans.* Especially, *S. sobrinus* is known to be more closely related to the prevalence of caries than *S*. *mutans.*

Herein, *"Streptococcus pneumoniae"* is a gram-positive bacterium and a species of streptococcus showing hemolysis. The strain does not cause symptoms in healthy persons, but the strain is a main cause of pneumonia and meningitis in children and the elderly with weak immunity and causes the upper part respiratory diseases.

Herein, *"Streptococcus pyogenes"* is a pyogenic streptococcus and is a gram-positive aerobic bacterium. *S*. *pyogenes* inhabits the throat, genital mucosa, rectum, and skin, and may cause various diseases, such as streptococcal pharyngitis, rheumatoid fever, rheumatoid heart disease, and scarlet fever, and causes the upper respiratory tract.

Herein, *"Escherichia coli"* is a representative gram-negative pathogenic bacterium, and the infection with *E*. *coli* by eating food containing *E. coli* or touching an animal infected with *E. coli* causes abdominal cramps, nausea, vomiting, and the like.

Herein, *"Staphylococcus aureus"* is a representative gram-positive pathogenic bacterium and a causative bacterium of pyogenic diseases and food poisoning. *S*. *aureus* is present in the nose, respiratory system, and skin, and food poisoning caused by *S. aureus* is a toxin-type food poisoning caused by ingestion of toxins produced by bacteria growing in food.

Herein, the antifungal activity may be an antifungal activity for *Candida albicans,* but is not limited thereto.

Herein, *"Candida albicans"* is an opportunistic pathogenic fungus that is a common member of the human gut flora. The strain is detected in the gastrointestinal tract and mouth in 40-60% of healthy adults, but causes Candidiasis by becoming pathogenic in immunocompromised individuals. Candidiasis is the third or fourth most frequent nosocomial infection worldwide, causing not only skin and genital infections, but also oral and esophageal infections, wherein systemic infections are treated with amphotericin B, echinocandin, or fluconazole.

The novel *S. salivarius* G7 (KCCM13161P) strain of the present disclosure has 4 to 8 times higher antibacterial effect compared with conventional *Streptococcus salivarius* K12 and *Streptococcus salivarius* ATCC 7073 (type strain).

The novel *S. salivarius* G7 (KCCM13161P) strain of the present disclosure has a higher halitosis suppressive effect compared with conventional *Streptococcus salivarius* K12 and *Streptococcus salivarius* ATCC 7073 (type strain). The *S. salivarius* G7 (Accession No. KCCM13161P) culture of the present disclosure can significantly suppress halitosis due to oral diseases by reducing the concentration of hydrogen sulfide (H₂S) and emulsified methyl (CH₃SH), which are volatile sulfur compounds causing halitosis in the *P*. *gingivalis* culture.

The present disclosure provides a composition for prevention or alleviation of an oral disease, the composition containing at least one of: the novel *Streptococcus salivarius* strain (*S. salivarius* G7, KCCM13161P) having antibacterial, antifungal, and antiinflammatory activities; a lysate thereof; a concentrate thereof; a dried product thereof; a culture thereof; an extract thereof; a thermally treated culture dried product thereof; and a fraction thereof.

The oral disease may be at least one selected from the group consisting of gingivitis, periodontitis, dental caries, halitosis, sensitive teeth, oral candidiasis, upper respiratory tract disease, and periapical disease, but is not limited thereto.

The oral disease may be caused by infection with pathogens or fungi in the oral cavity or upper respiratory tract, and the composition of the present disclosure may prevent or alleviate the oral diseases through antibacterial and antiinflammatory activities.

The composition for prevention or alleviation of the oral disease can be commercialized as one selected from the group consisting of toothpaste, gargling products, gums, candies, caramels, jellies, oral sprays, oral ointments, mouthwashes, mouthwashes, and tooth whitening agents, but is not limited thereto.

The present disclosure provides an oral probiotics composition containing a novel *Streptococcus salivarius* (*S. salivarius* G7, KCCM13161P) strain or a culture thereof having the antibacterial, antifungal, and antiinflammatory activity. The oral probiotics composition may be at least one formulation selected from the group consisting of tablets, capsules, a pill, granules, liquids, powders, flakes, pastes, syrups, gels, jellies, gums, and candies, but is not limited thereto. Examples of a carrier, an excipient, and a diluent that may be contained in the probiotics may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and a mineral oil. The probiotics composition, when formulated as a preparation, may be formulated using a diluent or an excipient, such as a filler, an extender, a binder, a wetting agent, a disintegrant, a surfactant, a sweetening agent, or an acidifier, which are commonly used.

The present disclosure provides a pharmaceutical composition for prevention or treatment of an oral disease, the composition containing at least one of: the novel *Streptococcus salivarius* strain (*S. salivarius* G7, KCCM13161P) having antibacterial, antifungal, and antiinflammatory activities; a lysate thereof; a concentrate thereof; a dried product thereof; a culture thereof; an extract thereof; a thermally treated culture dried product thereof; and a fraction thereof.

The oral disease may be at least one selected from the group consisting of gingivitis, periodontitis, dental caries, halitosis, sensitive teeth, oral candidiasis, upper respiratory tract disease, and periapical disease.

The novel *S. salivarius* G7 (KCCM13161P) strain, a lysate thereof; a lysate thereof; a concentrate thereof; a dried production thereof, a culture thereof, an extract thereof, a thermally treated culture dried product thereof, and a fraction thereof may be added in a content of preferably 0.001-50 wt%, more preferably 0.001-40 wt%, and most preferably 0.001-30 wt% to the total weight of the entire pharmaceutical composition.

The pharmaceutical composition may be formulated in an oral dosage form, such as a powder, granules, a tablet, a capsule, a suspension, an emulsion, a syrup, a liquid, or an aerosol, and in the form of an externally-applied preparation, according to a common method for each form. Examples of solid preparations for oral administration include a tablet, a pill, a powder, granules, a capsule, and the like, and these solid preparations may be prepared by mixing with at least one excipient, for example, starch, calcium carbonate, sucrose or lactose, and gelatin. In addition to simple excipients, lubricants, such as magnesium stearate and talc, may be used.

The dose of the pharmaceutical composition of the present disclosure may vary depending on the age, sex, and body weight of a subject to be treated, a particular disease or pathological condition to be treated, severity of a disease or pathological condition, route of administration, and determination of a prescriber. The determination of the dose based on these factors is within the level of a person skilled in the art, and the general dose is in the range of approximately 1×10⁶ CFU/day to 1×10¹¹ CFU/day. A preferable dose is 1×10⁸ CFU/day to 1×10¹¹ CFU/day, and a more preferable dose is 1×10⁸ CFU/day to 1×10¹⁰ CFU/day. The administration may be carried out once or several times in divided doses per day. The dose is not intended to limit the scope of the present disclosure in any aspect. The pharmaceutical composition of the present disclosure may be administered to mammals, such as mice, livestock, and humans, through various routes.

The present disclosure provides a food composition for prevention or alleviation of an oral disease, the composition containing at least one of: the novel *Streptococcus salivarius* strain (*S. salivarius* G7, KCCM13161P) having antibacterial, antifungal, and antiinflammatory activities; a lysate thereof; a concentrate thereof; a dried product thereof; a culture thereof; an extract thereof; a thermally treated culture dried product thereof; and a fraction thereof.

In the health functional food, at least one of the novel *S. salivarius* G7 (KCCM13161P) strain, a lysate thereof; a lysate thereof; a concentrate thereof; a dried production thereof, a culture thereof, an extract thereof, a thermally treated culture dried product thereof, and a fraction thereof may be added in a content of preferably 0.001-50 wt%, more preferably 0.001-30 wt%, and most preferably 0.001-10 wt% to the total weight of the entire pharmaceutical composition.

The health functional food includes a form of a tablet, a capsule, a pill, or a liquid preparation, and examples of a food to which the extract of the present disclosure can be added include various kinds of foods, beverages, gums, teas, vitamin complexes, health functional foods, and others.

The present disclosure relates to a novel *S. salivarius* G7 (KCCM13161P) strain, a composition containing the same for prevention or alleviation of an oral disease, and oral probiotics, and specifically, the novel *S*. *salivarius* G7 (KCCM13161P) strain can prevent, treat, and alleviate periodontal diseases through strong antibacterial and antiinflammatory activities against periodontal disease-causing strains and suppress halitosis caused by oral diseases, thereby contributing to oral health improvement.

### BRIEF DESCRIPTION OF THE DRAWINGS

The above and other aspects, features and advantages of the present disclosure will be more apparent from the following detailed description taken in conjunction with the accompanying drawings.
FIG. 1 shows *Streptococcus salivarius* G7 (Accession No. KCCM13161P) 16S rRNA sequence (SEQ ID NO: 1).
FIG. 2 shows a phylogenetic tree for *Streptococcus salivarius* G7 (Accession No. KCCM13161P).
FIG. 3 shows antibacterial activity experiment results of *Streptococcus salivarius* G7 (Accession No. KCCM13161P) against the gingivitis-causing bacteria *A. actinomycetemcomitans, A. israelii,* and *A. naeslundii* and the periapical inflammation-causing bacterium *E. faecalis.*
FIG. 4 shows antibacterial activity experiment results of *Streptococcus salivarius* G7 (Accession No. KCCM13161P) against the periodontitis-causing bacteria *F. nucleatum, P. gingivalis, P. intermedia,* and *P. nigrescens.*
FIG. 5 shows antibacterial activity experiment results of *Streptococcus salivarius* G7 (Accession No. KCCM13161P) against the dental caries-causing bacteria *A*. *viscosus, S. mutans,* and *S. sobrinus,* and the upper respiratory disease-causing bacteria *S. pneumoniae* and *S. pyogenes.*
FIG. 6 shows antibacterial activity experiment results of *Streptococcus salivarius* G7 (Accession No. KCCM13161P) against the representative gram-negative bacterium *E. coli* and gram-positive bacteria *S. aureus.*
FIG. 7 shows antifungal activity experiment results of *Streptococcus salivarius* G7 (Accession No. KCCM13161P) against the oral candidiasis-causing fungus *C. albicans.*
FIG. 8 confirms the virulence of a cell lysate containing cell surface substances of *S. salivarius* G7 (Accession No. KCCM13161P) of the present disclosure. A. TNF-α mRNA expression, B. IL-8 mRNA expression, C. TNF-α production, D. IL-8 production
FIG. 9 is a graph showing the effect of *S. salivarius* G7 (Accession No. KCCM13161P) LTA of the present disclosure on inflammatory cytokines induced by *P. gingivalis* LPS. A. TNF-α mRNA expression, B. IL-8 mRNA expression, C. TNF-α production, D. IL-8 production
FIG. 10 is a graph showing the effect of *S. salivarius* G7 (Accession No. KCCM13161P) LTA of the present disclosure on inflammatory cytokines induced by *T. forsythia* LPS. A. TNF-α mRNA expression, B. IL-8 mRNA expression, C. TNF-α production, D. IL-8 production
FIG. 11 is a graph showing the effect of *S. salivarius* G7 (Accession No. KCCM13161P) LTA of the present disclosure on inflammatory cytokines induced by *F. alocis LTA.* A. TNF-α mRNA expression, B. IL-8 mRNA expression, C. TNF-α production, D. IL-8 production
FIG. 12 shows flow cytometry results of ability of *S. salivarius* G7 (Accession No. KCCM13161P) LTA of the present disclosure to inhibit LPS and LTA binding to cells. A. *P*. *gingivalis* LPS binding, B. *T. forsythia* LPS binding, C. *F. alocis* LTA binding
FIG. 13 illustrates graphs showing ability of *S. salivarius* G7 (Accession No. KCCM13161P) LTA of the present disclosure to inhibit the binding, to CD14 and LBP, of periodontitis-related bacterial substances. A, B, C. *inhibition of P. gingivalis, T. forsythia, and F. alocis* binding to CD14, D, E. F. inhibition of *P. gingivalis, T. forsythia, and F. alocis* binding to LBP
FIG. 14 illustrates graphs showing the effect of *S. salivarius* G7 (Accession No. KCCM13161P) culture of the present disclosure on the concentrations of the *P. gingivalis-derived* volatile sulfur compounds hydrogen sulfide (H₂S) and methyl emulsified (CH₃SH) .
FIG. 15 illustrates graphs showing the effect of *S. salivarius* G7 (Accession No. KCCM13161P) strain of the present disclosure on the concentrations of the *P. gingivalis-derived* volatile sulfur compounds hydrogen sulfide (H₂S) and methyl emulsified (CH₃SH).
FIG. 16 shows co-focal laser microscopic three-dimensional images of cariogenic biofilms according to the treatment with or without *S. salivarius* G7 (Accession No. KCCM13161P) strain of the present disclosure (green: live bacteria, red: dead bacteria).
FIG. 17 illustrates graphs showing the total number of bacteria and the numbers of *S. mutans and S. salivarius* G7 bacteria in biofilms depending on the concentration of treatment with *S. salivarius* G7 (Accession No. KCCM13161P) Strain of the present disclosure.

### DETAILED DESCRIPTION OF THE EXEMPLARY EMBODIMENTS

Hereinafter, preferable exemplary embodiments of the present disclosure will be described in detail. However, the present disclosure is not limited to exemplary embodiments described herein and may be embodied in other forms, and exemplary embodiments introduced herein are provided to sufficiently convey the spirit of the present disclosure.

### <Example 1: Preparation of Streptococcus salivarius strain>

*Streptococcus salivarius* G7 (accession No. KCCM13161P) of the present disclosure was isolated, identified, and characterized from subgingival samples of healthy Korean males, and registered in the Korean Collection for Oral Microbiology (KCOM, *Streptococcus salivarius* KCOM2137). The strain was confirmed to be particularly excellent in antibacterial activity, antiinflammatory activity, dental caries inhibitory activity, and halitosis inhibitory activity than the conventional *Streptococcus salivarius* and deposited with the Korea Microorganism Conservation Center (Korea Seed Culture Association) (Accession No. KCCM1316P, Deposit Date: 2023.04.21.).

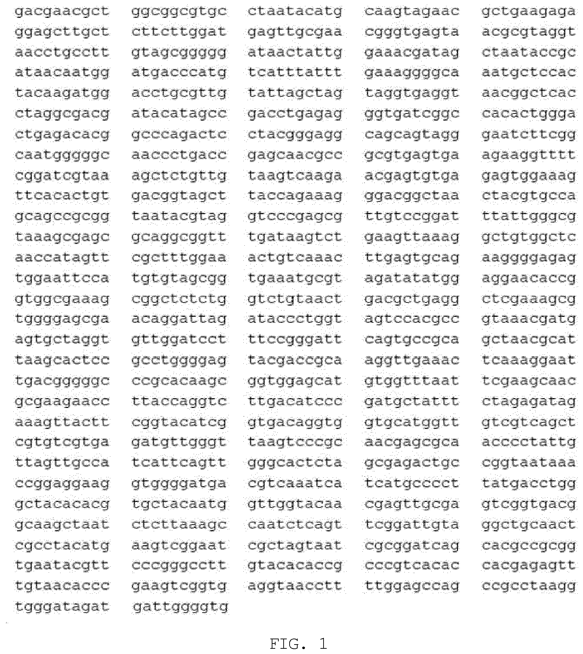

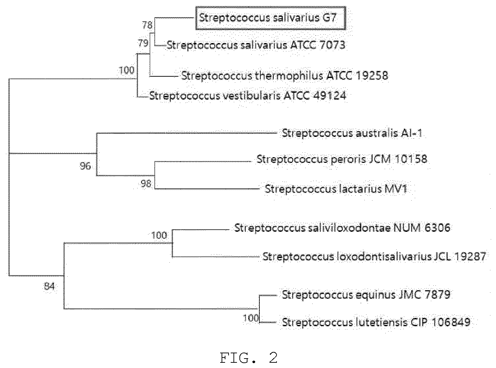

The 16S rRNA sequence (SEQ ID NO: 1) of *Streptococcus salivarius* G7 (accession No. KCCM13161P) of the present disclosure is shown in FIG. 1, based on which phylogenetic analysis was performed, and the results are shown in FIG. 2.

As comparative strains, *Streptococcus salivarius* K12 was isolated and identified from a commercially available product and *Streptococcus salivarius* ATCC 7073 (type strain) was purchased from the American Type Culture Collection (ATCC).

### <Example 2: Evaluation of antibacterial and antifungal activities>

### 2.1 bacterial culture

Oral bacteria for antibacterial activity test of the strains consisted of a total of 12 species of microorganisms, which were oral disease-related gram-positive and gram-negative bacteria, representative antibacterial test strains, and fungi, as shown in Table 1. As for dental caries-related bacteria, *Streptococcus mutans* ATCC 25175 and *S. sobrinus* ATCC 27607 were cultured using the trypticase soy broth (TSB; BD bioscience, San Jose, CA, USA), and *Actinomyces naeslundii* ATCC 12104 was cultured using the brain heart infusion (BHI; BD bioscience) liquid medium. As for gingival- and periodontal disease-related bacteria, *Actinomyces israelii* (ATCC 12102) and *Aggregatibacter actinomycetemcomitans* (ATCC 43718) were cultured using the brain heart infusion (BD bioscience), and *Fusobacterium nucleatum* (ATCC 25586), *Porphyromonas gingivalis* (ATCC 33277), *Prevotella intermedia* (ATCC 25611), and *Prevotella nigrescens* (ATCC 33563) were cultured using BHI liquid medium supplemented with hemin (1 µg/ml) and vitamin K (0.2 µg/ml), at 37°C under anaerobic conditions (H₂ 5%, CO₂ 10%, N₂ 85%). *Enterococcus faecalis* (ATCC 29212), which is an periapical inflammation-related bacterium, was cultured using BHI liquid medium at 37°C under anaerobic conditions (H₂ 5%, CO₂ 10%, N₂ 85%) . *Candida albicans* (ATCC 10231), which is a fungus, was cultured using TSB under aerobic conditions. *Streptococcus pyogenes (ATCC* 12344) and *S. pneumoniae (ATCC* 49416), which are upper respiratory tract disease-related bacteria, were cultured using Todd Hewitt broth (BD bioscience) under aerobic conditions. Last, *Staphylococcus* aureus(ATCC 23235) and *Escherichia coli*(ATCC 53868), which are representative gram-positive and gram-negative bacteria, were cultured using TSB under aerobic conditions. *S*. *salivarius* G7 (Accession No. KCCM13161P) of the present disclosure was cultured using M17 (BD bioscience) liquid medium.

**TABLE 1**

| Test strain | Related disease | Test strain | Related disease |
|---|---|---|---|
| *A. actinomycetemcomitans* | Adolescent periodontitis | *A. viscosus* | Dental caries |
| *A. israelii* | Gingivitis | *S. mutans* | |
| *A. naeslundii* | | *S. sobrinus* | |
| *F. nucleatum* | Periodontitis | *S. pneumoniae* | Upper respiratory tract disease |
| *P. gingivalis* | | *S. pyogenes* | |
| *P. intermedia* | | *C. albicans* | Oral candidiasis |
| *P. nigrescens* | | *E. coli* | Representative Pathogenic bacteria |
| *E. faecalis* | Periapical inflammation | *S. aureus* | |

### 2.2 Antibacterial activity test

Antibacterial activity test was performed using a method recommended by the Clinical & Laboratory Standard Institute. The bacteria cultured in the liquid media were centrifuged at 4,000 × g for 5 minutes, clean liquid media were added, and the bacteria were counted using a bacterial counting chamber. The measured bacterial suspension was adjusted using clean media to have 1.5 × 10⁶ cell/ml for aerobic bacteria and 1.0 × 10⁶ cell/ml for facultative anaerobic bacteria before use in the experiment.

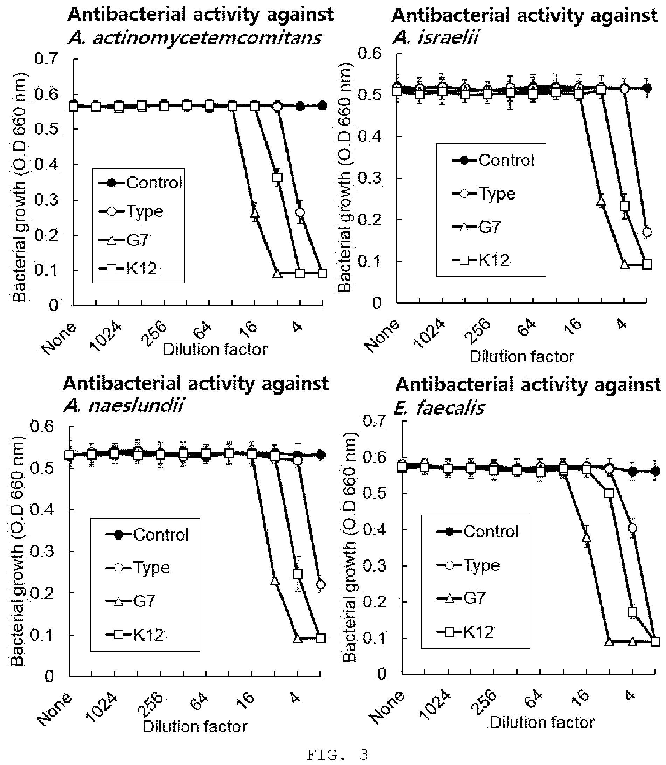

After 180 µl of each medium was added to a 96-well plate, 20 µl of G7 (Accession No. KCCM13161P) of the present disclosure and *S. salivarius* K12 and *S. salivarius* ATCC 7073, prepared in the first column, were added, and then each was diluted by two-fold serial dilution method, and cultured for 24 hours or 36 hours in an anaerobic incubator. Thereafter, the absorbance was measured at 660 nm using a spectrophotometer to analyze bacterial growth, which is shown in FIGS. 3 to 6.

As shown in FIG. 3, *S. salivarius* G7 (Accession No. KCCM13161P) according to the present disclosure showed superior antibacterial activity against the gingivitis-causing bacteria *A*. *actinomycetemcomitans, A. israelii, and A. naeslundii* and the periapical inflammation-related bacterium *E. faecalis* than *S. salivarius ATCC 7073* (type strain) or *S. salivarius K12,* which are conventionally known S. salivarius strains. *A. actinomycetemcomitans* causes gingivitis and may cause periodontitis in adolescents. *S. salivarius* G7 (Accession No. KCCM13161P) of the present disclosure inhibited the growth of *A. actinomycetemcomitans* in the 16-fold diluted culture medium and completely inhibited the growth in the 8-fold diluted culture medium. The strain of the present disclosure also inhibited the growth of *A*. *israelii* in the 8-fold diluted culture medium and completely inhibited the growth in the 4-fold diluted culture medium, and inhibited the growth of *A. naeslundii* in the 8-fold diluted culture medium and completely inhibited the growth in the 4-fold diluted culture medium. The strain of the present disclosure significantly inhibited the growth of *E. faecalis* in the 16-fold diluted culture medium and completely inhibited the growth in the 8-fold diluted culture medium. These results indicated that the strain of the present disclosure had 8 times or more antibacterial activity than conventional strains.

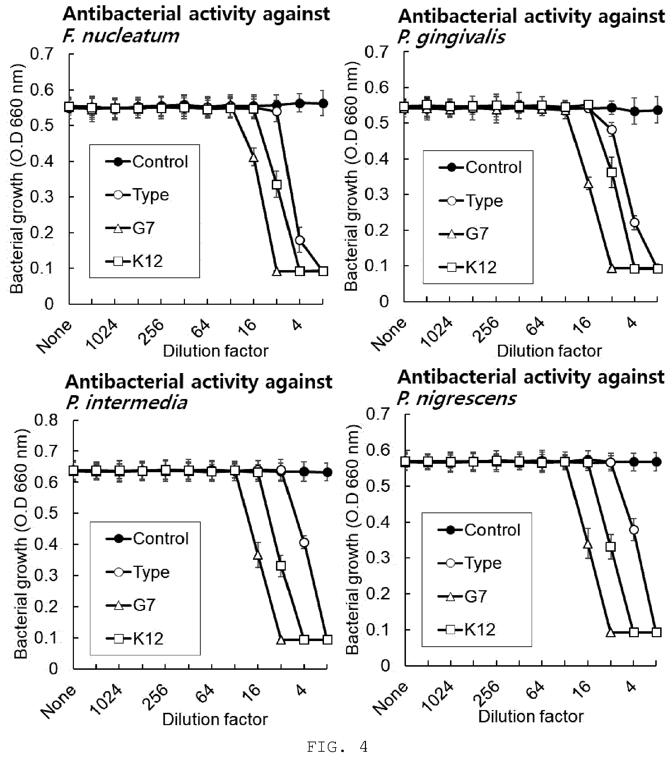

As shown in FIG. 4, *S. salivarius* G7 (Accession No. KCCM13161P) according to the present disclosure showed superior antibacterial activity against the periodontitis-causing bacteria *F. nucleatum, P. gingivalis, P. intermedia,* and *P. nigrescens* than *S. salivarius ATCC 7073* (type strain) or *S*. *salivarius K12,* which are conventionally known S. salivarius strains. *S. salivarius* G7 (Accession No. KCCM13161P) significantly inhibited the growth of *F. nucleatum, P. gingivalis, P. intermedia,* and *P. nigrescens in* the 16-fold diluted culture medium and completely inhibited the growth in the 8-fold diluted culture medium. These results indicated that the strain of the present disclosure had 8 times or more antibacterial activity than conventional strains.

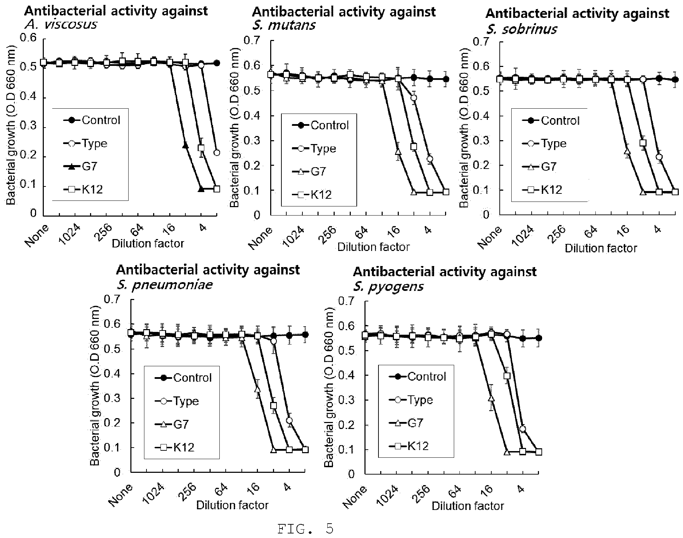

As shown in FIG. 5, *S. salivarius* G7 (Accession No. KCCM13161P) according to the present disclosure showed superior antibacterial activity against the dental caries-causing bacteria *A*. *viscosus, S. mutans* and *S. sobrinus* and the upper respiratory disease-causing bacteria *S*. *pneumoniae* and *S. pyogenes* than *S. salivarius* ATCC 7073 (type strain) or *S*. *salivarius* K12, which are conventionally known *S. salivarius* strains. *S. salivarius* G7 (Accession No. KCCM13161P) significantly inhibited the growth of *A. viscosus, S. mutans* and *S. sobrinus* and *S*. *pneumoniae* and *S. pyogenes* in the 16-fold diluted culture medium and completely inhibited the growth in the 8-fold diluted culture medium. These results indicated that the strain of the present disclosure had 8 times or more antibacterial activity than conventional strains.

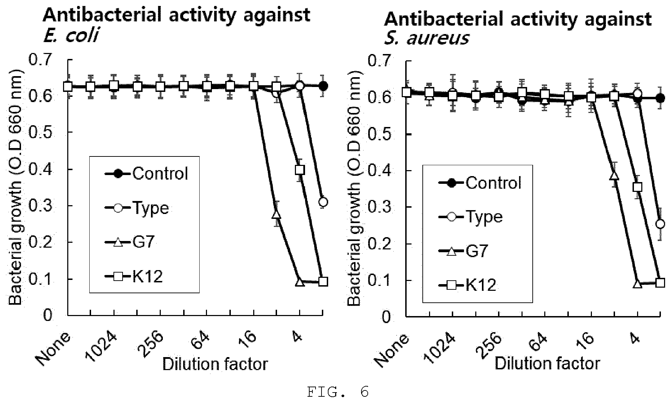

As shown in FIG. 6, *S. salivarius* G7 (Accession No. KCCM13161P) according to the present disclosure showed superior antibacterial activity against the representative gram-negative bacterium *E. coli* and gram-positive bacteria *S. aureus* than *S. salivarius* ATCC 7073 (type strain) or *S. salivarius* K12, which are conventionally known *S*. *salivarius* strains. *S. salivarius* G7 (Accession No. KCCM13161P) significantly inhibited the growth of *E. coli* and *S. aureus* in the 8-fold diluted culture medium and completely inhibited the growth in the 4-fold diluted culture medium. These results indicated that the strain of the present disclosure had 4 times or more antibacterial activity than conventional strains.

### 2.3 Antifungal activity test

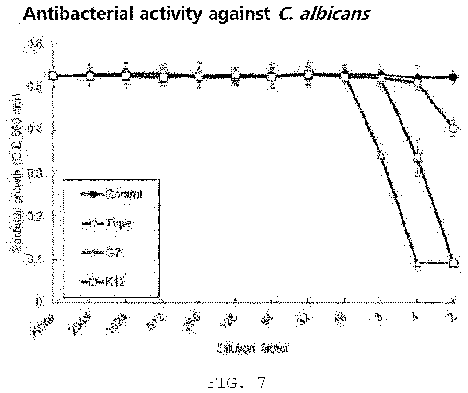

Antifungal activity test for the oral candidiasis-inducing fungus C. *albicans* was performed in the same manner as the antibacterial test, and the results are shown in FIG. 7.

As shown in FIG. 7, *S. salivarius* G7 (Accession No. KCCM13161P) according to the present disclosure showed superior antifungal activity against C. *albicans* than *S*. *salivarius* ATCC 7073 (type strain) or *S. salivarius* K12, which is a conventionally known *S. salivarius* strain. *S*. *salivarius* G7 (Accession No. KCCM13161P) significantly inhibited the growth of *C. albicans* in the 8-fold diluted culture medium and completely inhibited the growth in the 4-fold diluted culture medium. These results indicated that the strain of the present disclosure had 4 times or more antibacterial activity than conventional strains.

### <Example 3: Evaluation of antiinflammation activity>

The inflammation inhibitory effect of *S. salivarius* G7 (Accession No. KCCM13161P) of the present disclosure was examined using gingival fibroblasts. *Porphyromonas gingivalis*-derived lipopolysaccharide (LPS), *Tannerella forsythia*-derived lipopolysaccharide (LPS), or *Filifactor alocis*-derived lipoteichoic acid (LTA) were used as inflammation-inducing substances, as shown in Table 2 below.

**TABLE 2**

| Inflammation-inducing substance | Note |
|---|---|
| *Porphyromonas gingivalis lipopolysaccharide* | Periodontitis-related gram-negative bacteria |
| *Tannerella forsythia lipopolysaccharide* | |
| *Filifactor alocis lipoteichoic acid* | Periodontitis-related gram-positive bacteria |

*P. gingivalis* LPS, *T. forsythia* LPS, and *F. alocis* LTA initiate signal transduction, with starting the binding to TLR4 and TLR2 in oral cell membranes, respectively, and induce inflammation. CD14 and lipopolysaccharide-binding protein (LBP) are required to allow LPS or LTA to bind to TLR4 and TLR2. *S. salivarius* LTA can inhibit the inflammation-inducing activity of LPS and LTA by competitively inhibiting the binding of LPS and LTA of periodontitis bacteria to CD14 and LBP to prevent the binding to TLRs.

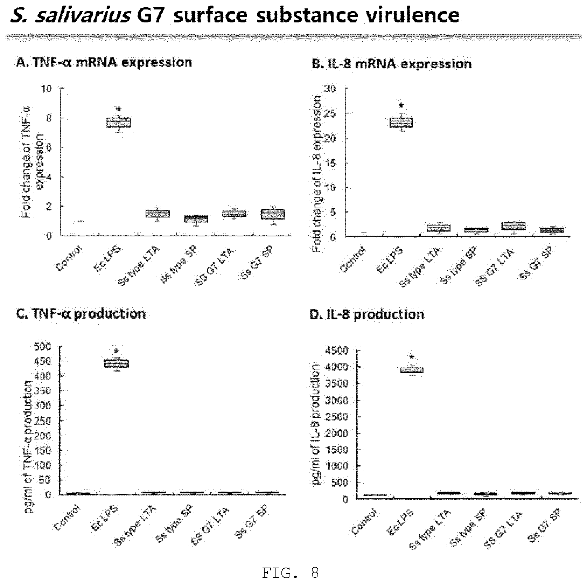

Gingival fibroblast cell line HGF-1 was inoculated into a 12-well polystyrene plate and cultured until the cells reached a confluency of 80%. Then, the medium was removed, and 1 ml of DMEM supplemented with 2% human serum (Sigma-Aldrich Co., San Jose, CA, USA) and penicillin/streptomycin (Hyclone) was added. Thereafter, the cell lysate obtained by disrupting *S. salivarius* G7 (Accession No. KCCM13161P) of the present disclosure using a sonicator was treated, and mixed with 100 µg of LPS or LPA as an inflammation-inducing substance, followed by culturing for 12 hours. Thereafter, the culture was analyzed for TNF-α and IL-8 by using ELISA kit. In addition, total RNA was extracted from the cells by using TRIzol (Invitrogen, Carlsbad, CA). The extracted RNA was measured for purity and concentration by using Nanodrop (Invitrogen), and cDNA was synthesized from 1 µg of total RNA by using Maxime RT premix (IntronBiotech., Gyeonggi), and inflammation-related cytokines were checked using the AB 7500 Real-time PCR instrument system (Applied Biosystems) and shown in FIGS. 8 to 11. Primer information of the inflammatory cytokines used in this experiment is shown in Table 3 below.

**TABLE 3**

| NC | Gene name | Direction | Nucleotide sequence |
|---|---|---|---|
| 2 | IL-8 | Forward | 5'-GTG AAG GTG CAG TTT TGC CA-3' |
| 3 | | Reverse | 5'-TCT CCA CAA CCC TCT GCA C-3' |
| 4 | TNF-α | Forward | 5'-CAG GGA CCT CTC TCT AAT CA-3' |
| 5 | | Reverse | 5'-AGC TGG TTA TCT CTC AGC TC-3' |
| 6 | GAPDH | Forward | 5'-GTG GTG GAC CTG ACC TGC-3' |
| 7 | | Reverse | 5'-TGA GCT TGA CAA AGT GGT CG-3' |

FIG. 8 confirms the virulence of the cell lysate containing cell surface substances of *S. salivarius* G7 (Accession No. KCCM13161P) of the present disclosure. The human gingival fibroblast cell line HGF-1 was treated with *E*. *coli-*derived lipopolysaccharides (Ec LPS), *S. salivarius* type strain (ATCC 7073)-derived lipoteichoic acid (Ss type LTA), *S. salivarius* type strain (ATCC 7073)-derived surface proteins (Ss type SP), present inventive *S. salivarius* G7 (Accession No. KCCM13161P)-derived LTA (SS G7 LTA), and present inventive *S*. *salivarius* G7 (Accession No. KCCM13161P)-derived SP (SS G7 SP), and mRNA and protein productions of TNF-α and IL-8 are shown in FIG. 8. As shown in FIG. 8, the mRNA and protein productions of TNF-α and IL-8 were increased by only the treatment with *E. coli-*derived LPS (Ec LPS), and LTA or SP of *S. salivarius* species did not act as a virulence factor.

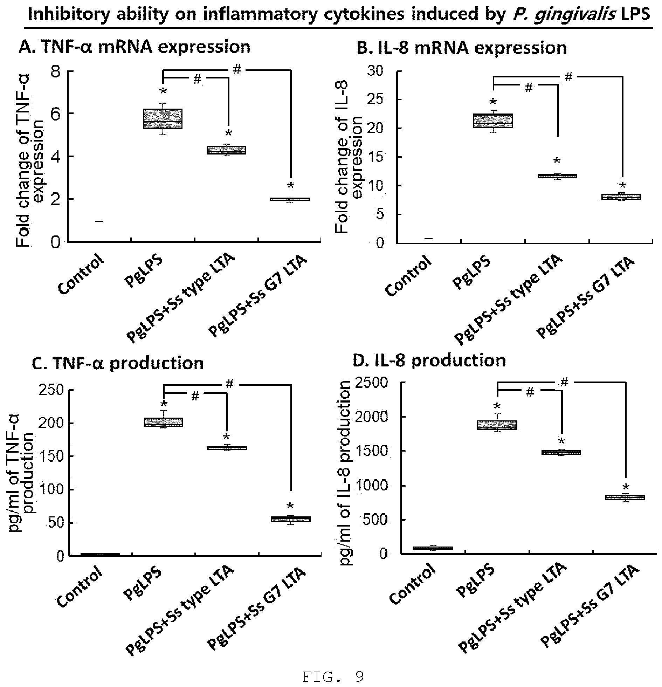

FIG. 9 is a graph showing the effect of *S. salivarius* G7 (Accession No. KCCM13161P) LTA of the present disclosure on inflammatory cytokines induced by *P. gingivalis* LPS. As shown in FIG. 9, the treatment of gingival fibroblast cell line HGF-1 with *P. gingivalis* LPS (Pg LPS) increased the mRNA and protein productions of TNF-α and IL-8, and the co-treatment with *S. salivarius* type strain and *S. salivarius* G7 (Accession No. KCCM13161P) LTA inhibited the mRNA and protein productions of TNF-α and IL-8. In particular, *S*. *salivarius* G7 (Accession No. KCCM13161P) LTA of the present disclosure significantly inhibited the mRNA and protein productions of TNF-α and IL-8 compared with the *S. salivarius* type strain.

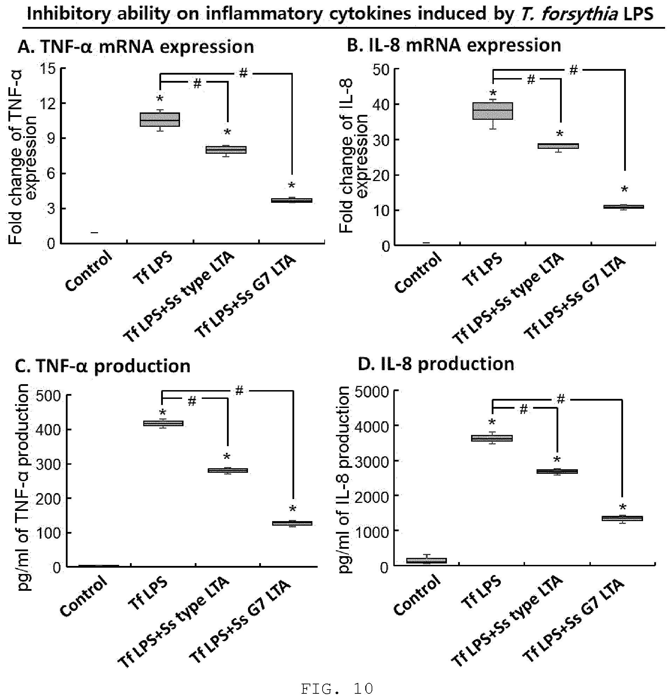

FIG. 10 is a graph showing the effect of *S. salivarius* G7 (Accession No. KCCM13161P) LTA of the present disclosure on inflammatory cytokines induced by *T. forsythia* LPS. As shown in FIG. 10, the treatment of gingival fibroblast cell line HGF-1 with *P. gingivalis* LPS (Pg LPS) increased the mRNA and protein productions of TNF-α and IL-8, and the co-treatment with *S. salivarius* type strain and *S. salivarius* G7 (Accession No. KCCM13161P) LTA inhibited the mRNA and protein productions of TNF-α and IL-8. In particular, *S*. *salivarius* G7 (Accession No. KCCM13161P) LTA of the present disclosure significantly inhibited the mRNA and protein productions of TNF-α and IL-8 compared with the *S. salivarius* type strain.

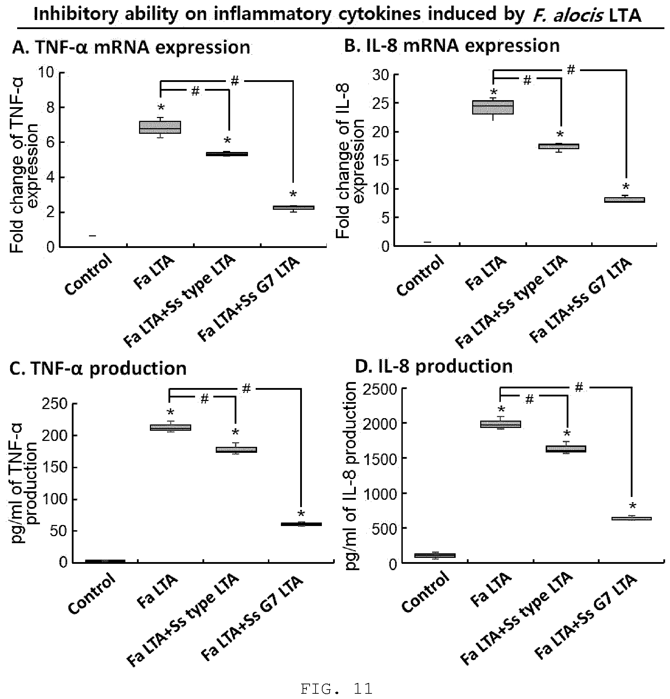

FIG. 11 is a graph showing the effect of *S. salivarius* G7 (Accession No. KCCM13161P) LTA of the present disclosure on inflammatory cytokines induced by *F. alocis LTA.* As shown in FIG. 11, the treatment of gingival fibroblast cell line HGF-1 with F. *alocis* LTA (FaLTA) increased the mRNA and protein productions of TNF-α and IL-8, and the co-treatment with *S. salivarius* type strain and *S. salivarius* G7 (Accession No. KCCM13161P) LTA inhibited the mRNA and protein productions of TNF-α and IL-8. In particular, *S*. *salivarius* G7 (Accession No. KCCM13161P) LTA of the present disclosure significantly inhibited the mRNA and protein productions of TNF-α and IL-8 compared with the *S. salivarius* type strain.

The above results confirmed that *S. salivarius* G7 (Accession No. KCCM13161P) of the present disclosure can inhibit the inflammation induced by periodontitis-causing bacteria.

### <Example 4: Evaluation of ability to inhibit binding of substance of periodontitis-related bacteria>

### 4.1 Flow cytometry

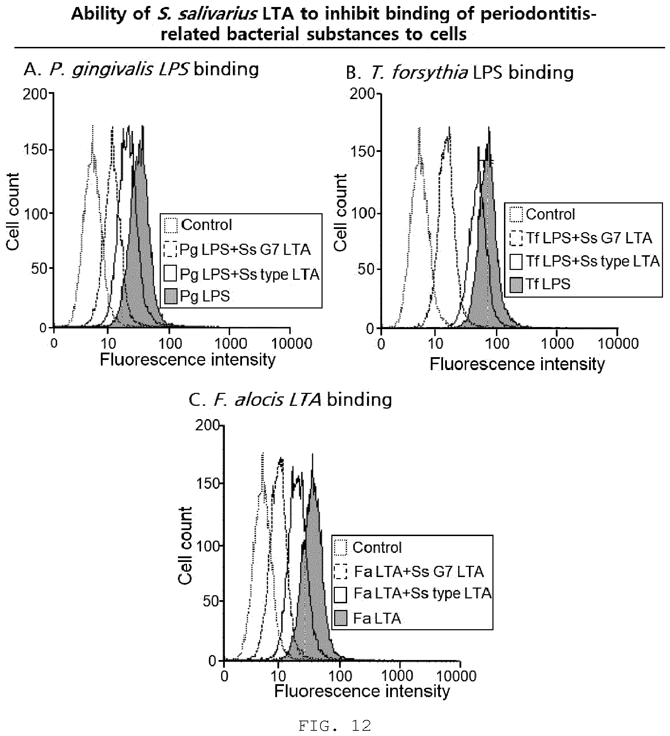

It was evaluated on the basis of the above results whether *S. salivarius* G7 (Accession No. KCCM13161P) LTA of the present disclosure could inhibit the binding of LPS or LTA derived from periodontitis-inducing bacteria to cells. A fluorescent substance was attached to *P. gingivalis* LPS, *T. forsythia* LPS and *F. alocis* LTA, and then the human gingival fibroblast cell line HGF-1 was treated with *S*. *salivarius* type strain (ATCC 7073)-derived LTA (Ss type LTA) or *S. salivarius* G7 of the present disclosure (Accession No. KCCM13161P ) LTA together was treated with human gingival fibroblast cell line HGF-1 alone or together and cultured, and then the degree of fluorescence of the cells was checked by flow cytometry and shown in FIG. 12.

LPS (1 mg) derived from *P. gingivalis* and *T. forsythia* and LTA derived from *F. alocis* were allowed to react with cold sodium metaperiodate at 4°C for 30 minutes, and then a reaction liquid was added to standard RC dialysis membrane tubing (Spectrum Laboratories Inc., Ranch Dominaquez, CA, USA) and dialyzed in 100 mM sodium acetate (pH 5.5) for 12 hours. The solution containing LPS and LTA was mixed with 10 mM Alexa FluorTM-488 hydrazide (Invitrogen, Carlsbad, CA, USA) prepared by dissolving in 200 mM KCl, and the mixture was left in a coupling buffer (0.1 M sodium phosphate, 0.15 M NaCl, pH 7.2) at room temperature for 6 hours, subjected to 3 rounds of dialysis in 1 L of endotoxin-free water for 12 hours, and then fluorescently labeled LPS and LTA were transferred to new tubes and stored at -20°C.

Thereafter, a human monocytic cell line THP-1 was treated with Alexa FluorTM 488-labeled LPS or LTA at 37°C for 1 hour in the presence or absence of various concentrations of *S. salivarius* G7 surface extract. Then, the cells were centrifuged at 500 × g for 3 minutes in a CO₂ incubator and washed three times with cold Dulbecco's phosphate buffered saline (DPBS, pH 7.2). Thereafter, the binding of Alexa 488-labeled LPS and LTA to the cells was analyzed by flow cytometry (FACSCalibur, BD Biosciences, Sparks, MD, USA). Data was collected by counting 10,000 cells, and the binding of Alexa Fluor^{™} 488-labeled LPS and LTA was detected in the FL-1 channel, and the data were analyzed using CellQuest software (BD biosciences).

As shown in FIG. 12, the treatment with *P. gingivalis* LPS, *T. forsythia* LPS, and *F. alocis* LTA greatly increased the number of fluorescent cells, that is, LPS- and LTA-bound cells, and the co-culture with *S. salivarius* type strain (ATCC 7073)-derived LTA (Ss type LTA) or *S. salivarius* G7 (accession number KCCM13161P) LTA of the present disclosure significantly reduced the number of fluorescent cells. In particular, *S. salivarius* G7 (Accession No. KCCM13161P) LTA of the present disclosure significantly reduced the LPS- and LTA-bound cells compared with the *S. salivarius* type strain.

### 4.2 Analysis of CD14 and LBP binding inhibition

In order for *P. gingivalis* LPS, *T. forsythia* LPS, or *F. alocis* LTA to induce inflammatory cytokines through TLR4 and TLR2, respectively, LPS and LTA need to bind to CD14 and LBP of the oral cell membrane. It was identified whether *S. salivarius* LTA competitively inhibited the binding of LPS and LTA of periodontitis bacteria to CD14 and LBP.

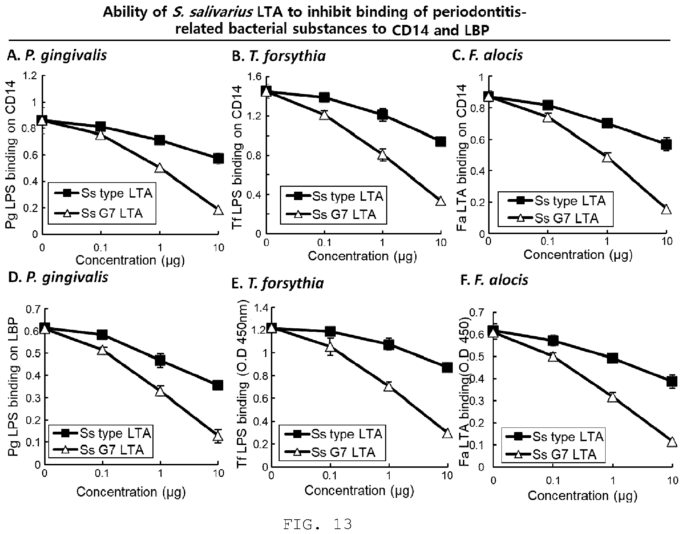

The fluorescent substance-attached *P. gingivalis* LPS, *T. forsythia* LPS and *F. alocis* LTA were cultured with CD14 or LBP protein in the presence of *S. salivarius* type strain (ATCC 7073)-derived LTA (Ss type LTA) or *S. salivarius* G7 (Accession No. KCCM13161P) LTA of the present disclosure at different concentrations, and the degree of binding was measured, and the results are shown in FIG. 13.

Anti-LBP mAb (MAB870, R&D systems, Minneapolis, MN, USA) and Anti-CD14 Ab (AF982, R&D systems) were dissolved in DPBS to a concentration of 1 µg/ml, and antibodies (50 ng/well) were plated on EIA plates (Corning Co., Corning, NY, USA) and then coated at 4°C for 12 hours. The antibody-coated EIA plates were washed 5 times with phosphate buffer solution (PBST) containing 0.1% tween 20, and DPBST containing 1% bovine serum albumin (BSA) was dispensed, and then the antibodies were inactivated by shaking for 2 hours under mild shaking conditions. Thereafter, recombinant human LBP (rhLBP, R&D system) and human CD14 (rhCD14, R&D system) were dissolved in DPBS at a concentration of 2 µg/ml and added to the wells of the antibody-coated EIA plates. The plates were left at room temperature for 4 hours, washed 5 times with PBST, and then the prepared wells were treated with biotin-labeled LPS or LTA and *S. salivarius* LTA alone or in combination and left at room temperature for 2 hours. To measure biotin-labeled LPS and LTA bound to rhLBP and rhCD14, they were reacted with 50 µl of HRP-labeled streptavidin (1 µg/ml) in PBST containing 2% BSA for 1 hour. After the plates were washed 5 times with PBST, a 3,3',5,5'-tetramethylbenzidine (TMB) solution was added to the well, and then left at room temperature for 30 minutes, and an enzyme reaction was stopped by 1N sulfuric acid. The absorbance was measured at a wavelength of 450 nm by a microplate reader (Biotek, Winooski, VT, USA).

As shown in FIG. 13, as *S. salivarius* type strain (ATCC 7073)-derived LTA (Ss type LTA) or *S. salivarius* G7 (Accession No. KCCM13161P) LTA of the present disclosure increased, the binding of *P. gingivalis* LPS, *T. forsythia* LPS, or *F. alocis* LTA to CD14 or LBP decreased. In particular, *S. salivarius* G7 (Accession No. KCCM13161P) LTA of the present disclosure significantly inhibited the binding of LPS or LTA to CD14 or LBP compared with the *S. salivarius* type strain.

### <Example 5: Evaluation of volatile sulfur compound inhibitory ability>

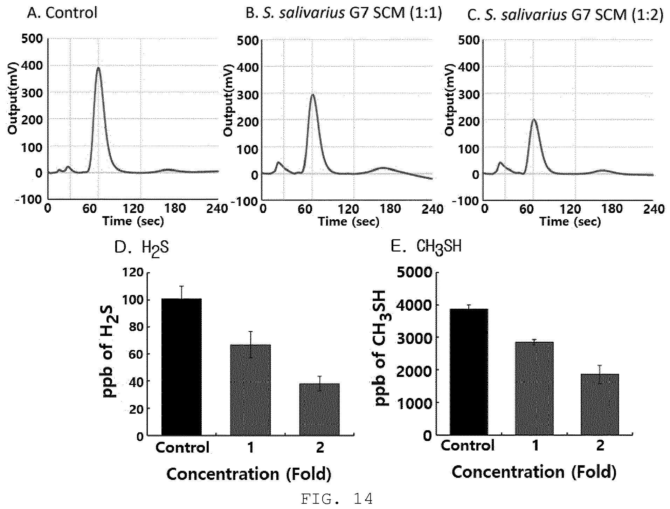

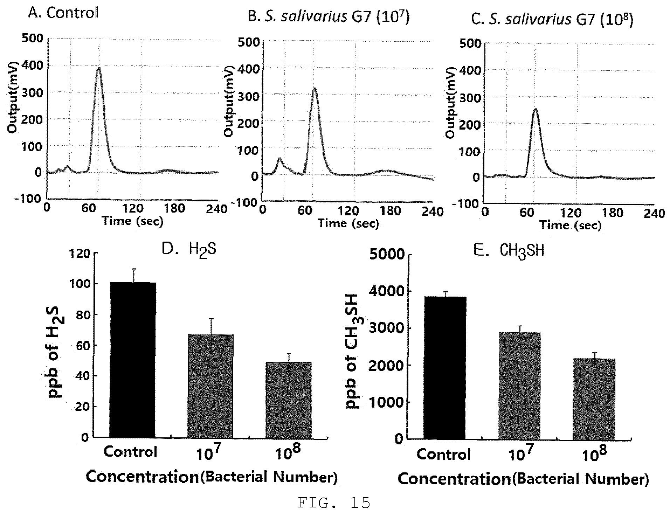

In order to examine whether *S. salivarius* G7 (Accession No. KCCM13161P) of the present disclosure had an anti-halitosis effect, an experiment was performed using a medium of *P*. *gingivalis* producing a volatile sulfur compound. After 1 ml of *P. gingivalis* (1 × 10⁸ cell/ml) was inoculated into 10 ml of a new medium and cultured for 48 hours, and then 300 µl of the bacteria culture was transferred to a clean 50 ml peripheral tube. Thereafter, 300 µl or 600 µl of the *S. salivarius* G7 (Accession No. KCCM13161P) culture of the present disclosure or 10⁷ or 10⁸ viable cells were added to the peripheral tube containing the *P. gingivalis* culture, and a medium was added to a final volume of 5 ml. Thereafter, the peripheral tube was shaken for 1 minute using a vertical shaker and left at room temperature for 5 minutes. Then, 1 ml of air directly above the surface of the solution was obtained using a 1 ml syringe, and the concentrations of hydrogen sulfide (H₂S) and methyl emulsified (CH₃SH) were measured using a gas chromatograph (OralchromaTM; Nissha FIS, Inc., Osaka, Japan). The results are shown in FIGS. 14 and 15.

As shown in FIG. 14, *S. salivarius* G7 (Accession No. KCCM13161P) culture of the present disclosure reduced the concentrations of hydrogen sulfide (H₂S) and methyl emulsified (CH₃SH), which are halitosis-causing volatile sulfur compounds in the *P. gingivalis* culture, in a concentration-dependent manner.

In addition, as shown in FIG. 15, *S. salivarius* G7 (Accession No. KCCM13161P) strain per se of the present disclosure reduced the concentrations of hydrogen sulfide (H₂S) and methyl emulsified (CH₃SH) in a concentration dependent manner.

The above results confirmed that *S. salivarius* G7 (Accession No. KCCM13161P) of the present disclosure could effectively suppress halitosis produced by *P. gingivalis.*

### <Example 6: Evaluation of biofilm formation inhibition>

### 6.1 Biofilm formation inhibitory effect

It was evaluated whether *S. salivarius* G7 (Accession No. KCCM13161P) of the present disclosure substantially inhibited biofilm formation. The saliva from 10 adults without periodontitis was collected, mixed, and divided into two tubes. First, in order to prepare a saliva-coated plate, an equivalent amount of a phosphate buffer solution was added into one of the tubes, followed by centrifugation at 6,000 × g for 10 minutes at 4°C, and the supernatant was transferred to a clean tube. Salivary bacteria were prepared by adding an equivalent amount of BHI medium to the saliva mixture in the other tube, followed by centrifugation at 2,000 × g for 10 minutes, and the supernatant was transferred to a clean tube.

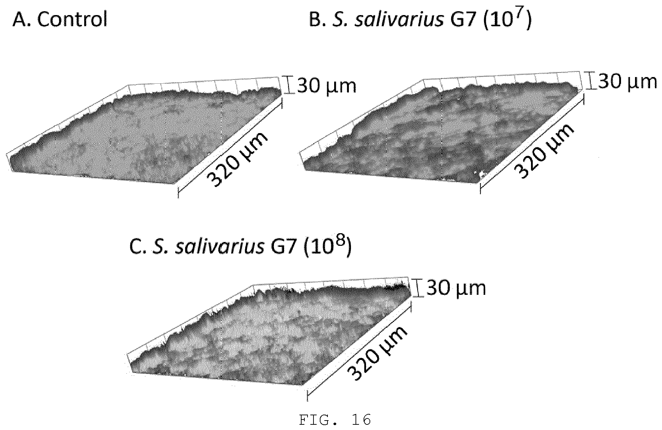

A procedure of dispensing 200 µl of saliva for coating into an 8-well glass plate and drying the saliva in an oven at 40°C was repeated 10 times. The saliva-coated plate prepared above was placed into a UV sterilizer, followed by sterilization. Thereafter, in order to form a cariogenic biofilm on 10 ml of salivary bacterial suspension containing 2% sucrose, 100 µl of *S. mutans* (1 × 10⁸ cell/ml) was added, and then 400 µl and 1 ml were added to an 8-well glass plate and a 12-well polystyrene plate, respectively, and cultured in aerobic conditions. Thereafter, a control group was cultured for 7 days by replacing the medium using only BHI medium containing 2% sucrose every day, and experimental groups were cultured by inoculating *S. salivarius* G7 (Accession No. KCCM13161P) of the present disclosure at two concentrations to include 10⁷ and 10⁸ strain cells in BHI containing 2% sucrose and then culturing for 7 days while changing the medium. Thereafter, in order to examine the amount of the biofilm, the 8-well plate was stained using a Bacterial live/dead staining kit (Invitrogen) and 3D-observed using a confocal laser microscope, and the results are shown in FIG. 16.

As shown in FIG. 16, the saliva strains and *S. mutans* formed a biofilm in combination on the saliva-coated plate, and when *S. salivarius* G7 (Accession No. KCCM13161P) of the present disclosure was added thereto, the biofilm was loosened while the number of dead biofilm-forming strains increased.

### 6.2 Measurement of number of bacteria in biofilm

The total number of bacteria (oral streptococci) and the numbers of bacteria of *S. mutans* and *S. salivarius* G7 (Accession No. KCCM13161P) in the formed biofilm were measured. In the above, 1 ml of DPBS was added to the 12-well plate to physically separate the cultured biofilm. This was centrifuged at 5,000 × g for 1 minute to obtain a bacterial precipitate, and thereafter, total DNA was extracted from the bacterial precipitate by using a G-spin Genomic DNA extraction kit (iNtRON Biotechnology, Gyeonggi, Korea), and then the bacterial quantification was conducted using a real-time gene amplifier.

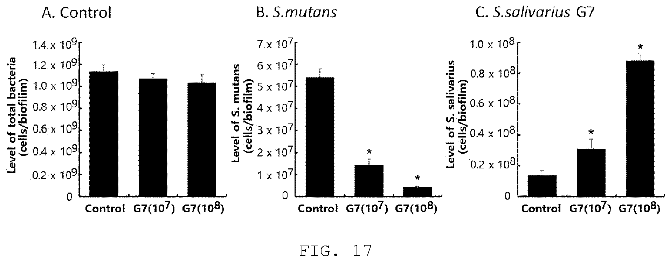

Real-time genetic analysis was performed on corresponding standard quantitative DNA and samples. TB green premix (Takara Co., Tokyo, Japan), ROX II dye, Primers, and DNA were mixed and pre-heated at 94°C for 5 minutes, followed by 40 cycles of 95°C for 10 seconds (denaturation stage), 60°C for 10 seconds (annealing stage), and 72°C for 33 seconds (extension stage). The product melting curve was used to examine non-specific binding. A standard curve for the Ct value and the amount of bacteria was formed using the standard quantitative DNA, and based on the standard curve, the number of bacteria was calculated using the Ct value of sample DNA, and the results are shown in FIG. 17. The primers used are shown in Table 4.

**TABLE 4**

| **NO** | **Name** | **Direction:** | **Nucleotide sequence** |
|---|---|---|---|
| 8 | *Oral streptococci* | Forward | 5-AAG CAA CGC GAA GAA CCT TA-3' |
| 9 | *Oral streptococci* | Reverse | 5'-GTC TCG CTA GAG TGC CCA AC-3' |
| 10 | *S*. *mutans* | Forward | 5`-CTA CAC TTT CGG GTG GCT TG-3' |
| 11 | *S. mutans* | Reverse | 5'-GAA GCT TTT CAC CAT TAG AAG CTG-3' |
| 12 | *S. salivarius* | Forward | 5′-TTG CCA CAT CTT CAC TCG CTT- 3′ |
| 13 | *S. salivarius* | Reverse | 5′-GTT TAG TAG CAA CTT CTG GCT T-3′ |

As shown in FIG. 17, the number of bacteria of *S*. *salivarius* G7 (Accession No. KCCM13161P) in the biofilm increased with an increasing amount of treatment thereof, but the number of bacteria of *S*. *mutans,* a causative bacterium of dental caries, significantly decreased with an increasing amount of treatment of *S*. *salivarius* G7 (Accession No. KCCM13161P) of the present disclosure.

### [Accession Number]

Depository institution name: Korean Culture Center of Microorganism (KCCM)
Accession No: KCCM13161P
Deposit date: 20220421

### [Sequence Listing]

Sequence Listing Electronic File Attachment

## Claims

1. A novel *Streptococcus salivarius* strain KCCM13161P (*S*. *salivarius* G7, KCCM13161P) having antibacterial, antifungal, and antiinflammatory activities and dental caries inhibitory activity.

2. The strain of claim 1, wherein the antibacterial activity is against at least one selected from the group consisting of *Aggregatibacter actinomycetemcomitans, Actinomyces israelii, Actinomyces naeslundii, Enterococcus faecalis, Fusobacterium nucleatum, Porphyromonas gingivalis, Prevotella intermedia, Bacteroides intermedius, Prevotella nigrescens, Streptococcus mutans, Streptococcus sobrinus, Streptococcus pneumoniae, Streptococcus pyogenes, Actinomyces viscosus, Escherichia coli,* and *Staphylococcus aureus.*

3. The strain of claim 1, wherein the antifungal activity is against *Candida albicans.*

4. A composition comprising at least one of: the strain of any one of claims 1 to 3; a lysate thereof; a concentrate thereof; a dried product thereof; a culture thereof; an extract thereof; a thermally treated culture dried product thereof; and a fraction thereof.

5. The composition of claim 4, wherein the composition is for use in the prevention or alleviation of an oral disease.

6. The composition of claim 5, wherein the oral disease is at least one selected from the group consisting of gingivitis, periodontitis, dental caries, halitosis, sensitive teeth, oral candidiasis, upper respiratory tract disease, and periapical disease.

7. The composition of claim 4, wherein the composition is commercialized as one selected from the group consisting of toothpastes, gargling products, gums, candies, caramels, jellies, oral sprays, oral ointments, mouth washes, mouth fresheners, and tooth whiteners.

8. An oral probiotics composition comprising the strain of any one of claims 1 to 3 or a culture thereof.

9. The oral probiotics composition of claim 7, wherein the oral probiotics composition is at least one formulation selected from the group consisting of tablets, capsules, pills, granules, liquids, powders, flakes, pastes, syrups, gels, jellies, gums, and candies.

10. A pharmaceutical composition comprising at least one of: the strain of any one of claims 1 to 3; a lysate thereof; a concentrate thereof; a dried product thereof; a culture thereof; an extract thereof; a thermally treated culture dried product thereof; and a fraction thereof.

11. The pharmaceutical composition of claim 10, wherein the pharmaceutical composition is for use in the prevention or alleviation of an oral disease

12. The pharmaceutical composition for use of claim 11, wherein the oral disease is at least one selected from the group consisting of gingivitis, periodontitis, dental caries, halitosis, sensitive teeth, oral candidiasis, upper respiratory tract disease, and periapical disease.

13. A health functional food composition, the composition comprising at least one of: the strain of any one of claims 1 to 3; a lysate thereof; a concentrate thereof; a dried product thereof; a culture thereof; an extract thereof; a thermally treated culture dried product thereof; and a fraction thereof.

14. The health functional food of claim 13, wherein the health functional food is for prevention or alleviation of an oral disease.

## Amended claims

### Amended claims in accordance with Rule 137(2) EPC.

1. A pharmaceutical composition for use in alleviating or treating an oral disease, the composition comprising at least one of: *Streptococcus salivarius* strain KCCM13161P (S. *salivarius* G7, KCCM13161P); a lysate thereof; a concentrate thereof; a dried product thereof; a culture thereof; an extract thereof; a thermally treated culture dried product thereof; and a fraction thereof, wherein the oral disease is oral candidiasis.

2. The pharmaceutical composition for use of claim 1, wherein the pharmaceutical composition has an antifungal activity against *Candida albicans.*

3. A pharmaceutical composition for use in suppressing halitosis, the composition comprising at least one of: *Streptococcus salivarius* strain KCCM13161P *(S. salivarius* G7, KCCM13161P); a lysate thereof; a concentrate thereof; a dried product thereof; a culture thereof; an extract thereof; a thermally treated culture dried product thereof; and a fraction thereof.

4. The pharmaceutical composition for use of claim 3, wherein the suppression of halitosis is performed by reducing the concentrations of hydrogen sulfide (H₂S) and methyl emulsified (CH₃SH).

5. The pharmaceutical composition for use of any one of claims 1 to 4, wherein the pharmaceutical composition is selected from the group consisting of toothpastes, gargling products, gums, candies, caramels, jellies, oral sprays, oral ointments, mouth washes, mouth fresheners, and tooth whiteners.
